Europäisches Patentamt

European Patent Office

Office européen des brevets

(11) Numéro de publication: **0 324 728**
**A1**

# DEMANDE DE BREVET EUROPEEN

(21) Numéro de dépôt: **89870008.3**

(22) Date de dépôt: **16.01.89**

(51) Int. Cl.⁴: **A 01 K 27/00**
A 01 K 29/00, A 61 D 17/00,
A 61 B 5/08, A 61 B 5/03

(30) Priorité: **15.01.88 BE 8800054**

(43) Date de publication de la demande:
**19.07.89 Bulletin 89/29**

(84) Etats contractants désignés: **ES GR**

(71) Demandeur: **S.A. AGRITRONICS N.V.**
**17, rue du Village**
**B-5854 Meux-la-Bruyère (BE)**

(72) Inventeur: **Cobben, Andrée**
**rue de Liernu 8**
**B-5854 Meux-la-Bruyère (BE)**

**Jaspar, Francis**
**rue de Liernu 8**
**B-5854 Meux-la-Bruyère (BE)**

**Mouthuy, Christian**
**172, avenue Victor Hugo**
**B-1410 Waterloo (BE)**

(74) Mandataire: **Van Malderen, Michel et al**
**p.a. Office van Malderen avenue J.S. Bach 22/43**
**B-1080 Bruxelles (BE)**

(54) Dispositif à sangles destiné à des animaux.

(57) Dispositif à sangles destiné notamment à maintenir confortablement et sans risques de blessures un dispositif de surveillance d'animaux.

Le dispositif est constitué d'une sangle avant (1) et d'une sangle arrière (3) comportant au moins partiellement une zone élastique formant ceinture en entourant l'abdomen de l'animal et par au moins une sangle transversale supérieure (5), et de préférence deux sangles transversales (supérieures 5 et 5'), reliant la sangle avant (1) et la sangle arrière (3).

Application notamment au maintien du dispositif de surveillance de l'activité musculaire annonçant le début de vêlage.

FIG.1

EP 0 324 728 A1

Description

## DISPOSITIF A SANGLES DESTINE A DES ANIMAUX

### Arrière-plan technologique

On sait que pour la surveillance de la parturition des animaux notamment, on a proposé d'équiper les animaux à surveiller (généralement des vaches) d'un dispositif de mesure de l'activité musculaire, contenu dans un boîtier, qui contient généralement de plus un émetteur de signaux (publication WO/-87/01573).

Les animaux ainsi équipés peuvent être laissés en pâture ou en étable. L'équipement complet comporte un récepteur et est conçu de manière à analyser les signaux du dispositif de mesure et à déclencher une alarme ou un signal transmis à l'éleveur, en temps opportun, avant la mise bas.

On a utilisé initialement une ceinture simple pour maintenir l'émetteur sur le dos de l'animal mais l'on s'est aperçu rapidement que cette ceinture ne permettait pas de maintenir convenablement en place le dispositif de mesure de manière continue et dans toutes les situations de la vie normale de l'animal.

En effet, la tension de la ceinture, en particulier pour des animaux gravides ne peut pas être exagérée. Il convient également d'éviter des blessures des parties sensibles de l'abdomen (pis des vaches notamment). Une simple ceinture ne permet donc pas de maintenir le dispositif de mesure sur le dos de l'animal, la ceinture ayant tendance à tourner ou à se déplacer, voire éventuellement tomber, d'autant plus que certains animaux se frottent contre des arbres, des clôtures, des murs, etc.

La demanderesse s'est aperçue également qu'un dispositif de mesure qui comporte un élément sensible agissant dans le sens longitudinal de déformation de la ceinture, pour fonctionner convenablement, doit être conçu de manière adéquate, à la fois pour pouvoir mesurer de manière précise l'activité musculaire et pour la transmettre vers un système d'analyse.

Une série d'autres considérations, notamment d'hygiène, de confort de l'animal, etc... entrent également en ligne de compte pour la mise au point d'un dispositif qui doit assurer le maintien de l'appareillage équipant l'animal et pour assurer la mesure et la transmission des signaux relatifs à l'activité musculaire.

De plus, il convient que les qualités de confort et d'hygiène notamment du dispositif permettent de maintenir l'appareillage de mesure sur l'animal pendant des périodes fort longues pouvant atteindre plusieurs semaines.

Ceci reste valable pour le maintien de tout autre objet ou équipement sur un animal.

Par le document US-A-4 676 198, on connaît un harnais destiné à maintenir un chien ou un autre animal en place, notamment à l'immobiliser dans un véhicule. Ce harnais comporte une sangle avant et une sangle arrière entourant le corps de l'animal qui sont reliées entre elles par une sangle transversale supérieure et une sangle transversale inférieure. De plus une sangle entourant le cou et le poitrail de l'animal ainsi qu'une laisse permettant d'attacher l'animal sont prévues. Toutes ces sangles sont réglables de manière à s'adapter à la taille de l'animal. Bien qu'adaptable, ce harnais ne pourrait être supporté pendant de longues périodes par l'animal car il est trop rigide. Un tel harnais n'est pas transposable pour le but recherché par l'invention, car la sangle entourant le cou et le poitrail de l'animal ainsi que la sangle transversale inférieure risque de blesser l'animal en des endroits particulièrement sensibles tels que le poitrail et le pis d'une vache par exemple.

### Objet de l'invention

La présente invention vise à fournir un dispositif à sangles convenant à l'usage indiqué et qui est en particulier bien adapté à la fonction de maintien d'un boîtier du type décrit ou d'un objet similaire et qui évite largement les inconvénients des solutions de l'art antérieur.

### Eléments caractéristiques du dispositif de l'invention

Le dispositif à sangles selon l'invention est essentiellement constitué par une sangle avant et une sangle arrière comportant au moins partiellement une zone élastique formant ceinture en entourant l'abdomen de l'animal et par au moins une sangle transversale supérieure, et de préférence deux sangles transversales supérieures, reliant la sangle avant et la sangle arrière.

Chaque sangle transversale est conçue à chaque extrémité pour comporter un moyen tel qu'une boucle ou une découpe lui permettant de recevoir par glissement la sangle arrière et la sangle avant. Les sangles transversales sont prévues pour former un angle déterminé (par exemple 45°) par rapport aux sangles arrière et avant, quand l'animal en est équipé et sont pourvues de moyens pour leur maintien en place.

La longueur de toutes les sangles est bien entendu adaptée à la morphologie de l'animal avec des possibilités de réglage.

Une forme d'exécution particulière du dispositif selon l'invention consiste à faire se rejoindre les deux extrémités de deux sangles transversales supérieures en une seule patte de manière à solidariser cette patte à un boîtier de mesure fixé à la sangle arrière. L'extrémité de la patte de jonction des deux sangles transversales comporte deux découpes rectangulaires dans le sens longitudinal de la patte qui permettent de maintenir le boîtier sur l'animal en enveloppant ce boîtier par la patte, ce qui protège l'animal d'éventuelles blessures dues aux frottements des arêtes du boîtier sur son corps.

Pour certaines races, il est apparu utile de prévoir sur la sangle avant, deux petites sangles en forme de bouclettes entourant chacune une patte avant de l'animal et contribuant au maintien en place de l'ensemble des sangles lors de leur utilisation sur l'animal.

La sangle arrière est par exemple destinée à transmettre l'activité musculaire vers le dispositif d'analyse. Le boîtier de ce dispositif est pourvu à ses deux extrémité d'ouvertures dans lesquelles peuvent coulisser les deux extrémités de la sangle arrière. Cette sangle est équipée également de dispositifs de réglage de tension bien connus.

La sangle arrière comporte comme caractéristique essentielle le fait qu'elle est constituée sur sa partie centrale au moins d'une bande élastique, tandis que de préférence les deux extrémités ne sont que très faiblement déformables élastiquement.

La zone de transition entre la partie élastique médiane et les extrémités est elle-même non élastique mais de largeur moindre que les parties adjacentes.

D'autres éléments caractéristiques et détails spécifiques de l'invention apparaîtront à la lecture de la description qui suit d'une forme d'exécution préférée en regard des dessins annexés.

**Brève description des dessins**

La figure 1 représente schématiquement une vue en perspective du dispositif de l'invention, pratiquement dans la position où il est utilisé, mais sans bouclage de ses éléments de fermeture; on a également représenté le boîtier d'un dispositif d'analyse.

La figure 2 représente une vue en détail d'une extrémité d'une sangle transversale indiquant la façon dont il est possible de la bloquer sur une sangle avant ou arrière.

La figure 3 représente une bouclette de fixation complémentaire visible à la figure 1.

La figure 4 représente un détail du montage de bouclettes pouvant être éventuellement montée sur une sangle avant afin d'assurer un meilleur maintien qui évite la rotation.

La figure 5 représente le détail de la fixation d'un dispositif solidarisable à une extrémité de la sangle arrière sur le boîtier d'un dispositif d'analyse.

Les figures 6a, 6b, 6c représentent les détails de l'emboîtage des éléments de la figure 5.

La figure 7 représente une vue en à plat de l'ensemble des deux sangles transversales se rejoignant en une seule patte qui constitue une forme d'exécution particulière de l'invention.

La figure 8 représente une vue partielle correspondant à l'élément de la figure 7. On a représenté en perspective le moyen de liaison de la patte de jonction des deux sangles transversales au boîtier de mesure.

**Description d'une forme d'exécution du dispositif de l'invention**

Le dispositif de l'invention est constitué d'une sangle avant 1, d'une sangle arrière 3 et de deux sangles transversales 5 et 5'. Il est possible de n'utiliser qu'une seule sangle transversale mais ceci n'est pas à conseiller.

Un équipement de détection de l'activité musculaire contenu dans un boîtier 9 ou un autre dispositif à maintenir est fixé sur la sangle arrière 3 par solidarisation de celle-ci avec le boîtier 9. Les sangles arrière 3 et avant 1 sont rendues solidaires par les deux transversales 5. La rotation du système est totalement empêchée si l'on souhaite par l'utilisation des bouclettes 13 qui peuvent servir, le cas échéant, de complément de fixation.

La sangle avant 1 n'exerçant qu'essentiellement une fonction de maintien est de conception largement quelconque et se termine à une extrémité par un dispositif 7 de bouclage dans lequel on peut faire glisser et bloquer l'autre extrémité de cette sangle.

La sangle arrière 3 est quant à elle destinée à maintenir le boîtier de mesure 9.

Les deux bouts de la sangle arrière 3 sont enfilés dans deux éléments solidaires ou faisant partie du boîtier de mesure 9.

Bien entendu, d'autres moyens de solidarisation, de préférence amovibles, sont également possibles.

Un réglage de la tension est possible à l'aide d'un ou de plusieurs dispositifs du type 11.

La sangle arrière 3 est constituée d'une partie élastique 3a, d'une partie 3b de dimension inférieure dans la largeur et de deux parties d'extrémité (3c et 3d). Les parties 3b, 3c et 3d sont constituées en un matériau pratiquement inélastique. Les parties 3c destinées à s'appliquer sur le flanc de l'animal sont pourvues de perforations qui assurent l'évaporation de la transpiration de l'animal.

Deux sangles transversales 5 et 5' sont glissées respectivement à gauche et à droite par rapport à l'animal quand il sera équipé du dispositif, sur les sangles avant 1 et arrière 3.

Sur la sangle avant 1, des bouclettes 13 et 13' destinées à entourer les pattes avant de l'animal peuvent servir de complement de fixation. Elles ne sont pas nécessaires cependant pour certaines races animales.

La sangle arrière 3 est de préférence constituée d'une bande de matière du type EPDM (qualité alimentaire) vulcanisé ou similaire qui est renforcée dans les parties pratiquement non élastiques 3b, 3c et 3d, par une toile textile incorporée dans le polymère au cours de la fabrication de la matière qui sera découpée ensuite. Bien entendu les autres parties du dispositif peuvent être réalisées dans la même matière.

On notera qu'en plus des perforations déjà décrites de la sangle arrière, de nombreuses autres perforations sont prévues dans les différentes sangles.

Le retrécissement 3b de la sangle arrière a pour effet, lorsqu'on tend celle-ci, qu'elle affecte par une auto-déformation une forme légèrement recourbée dans le sens transversal, en particulier dans la zone élastique 3a. L'élasticité et cette courbure protègent efficacement l'animal au niveau du pis ou au niveau de toute autre zone sensible.

On notera que les bouts 3e de la sangle arrière 3 (et ceci est également applicable à la sangle avant 1) se terminent en pointe éventuellement tronquée et que la partie 3e est à nouveau plus large que la partie qui lui est adjacente 3d. Ceci, de même que l'élargissement 3d limite la course des éléments de fixation 11.

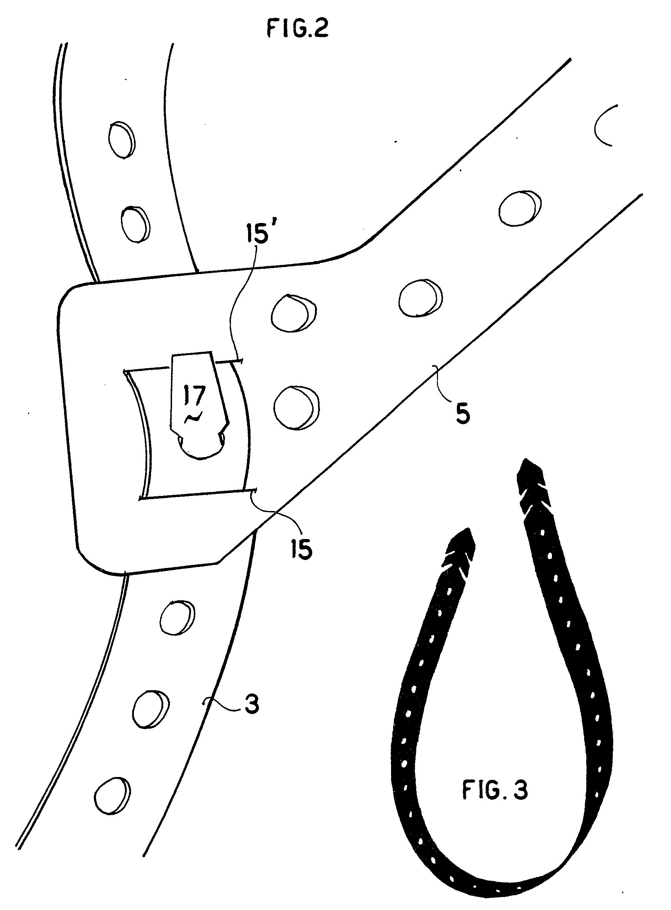

La figure 2 représente l'extrémité d'une sangle transversale 5. Elle comporte deux découpes 15 et 15′ dans lesquelles la sangle avant peut entièrement passer et dans laquelle les bouts de la sangle arrère 3 peuvent également glisser. Une ou plusieurs languettes de positionnement 17 découpée dans la matière de la sangle 5 peut servir à maintenir en position la sangle transversale par rapport aux sangles arrière et avant, en pénétrant dans des perforations correspondantes.

Le boîtier qui est destiné à mesurer en usage l'activité musculaire de l'animal, permet également de mesurer la tension dans la sangle et peut servir à indiquer par un signal, un réglage correct ou un réglage incorrect (aussi bien vers le haut que vers le bas) de cette tension et ceci tant lors de la mise en place qu'après cette mise en place.

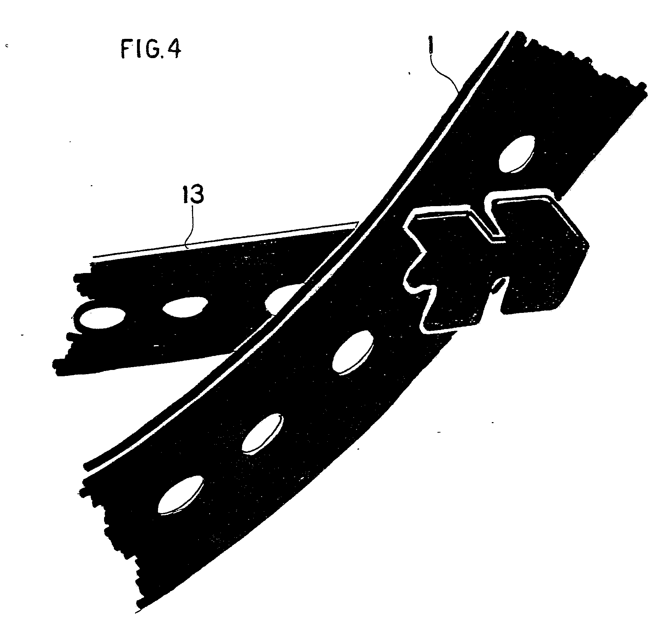

La forme d'une bouclette 13 est visible à la figure 3, un détail du montage de cette bouclette 13 sur une sangle avant 1 apparaît à la figure 4. On peut voir que la bouclette 13 se termine en forme de "sapin". Cette terminaison en "sapin" est engagée dans un des trous de la sangle avant 1. La bouclette passe entre les pattes de l'animal et l'autre extrémité est à nouveau engagée dans un des trous de la sangle avant permettant ainsi un réglage du système.

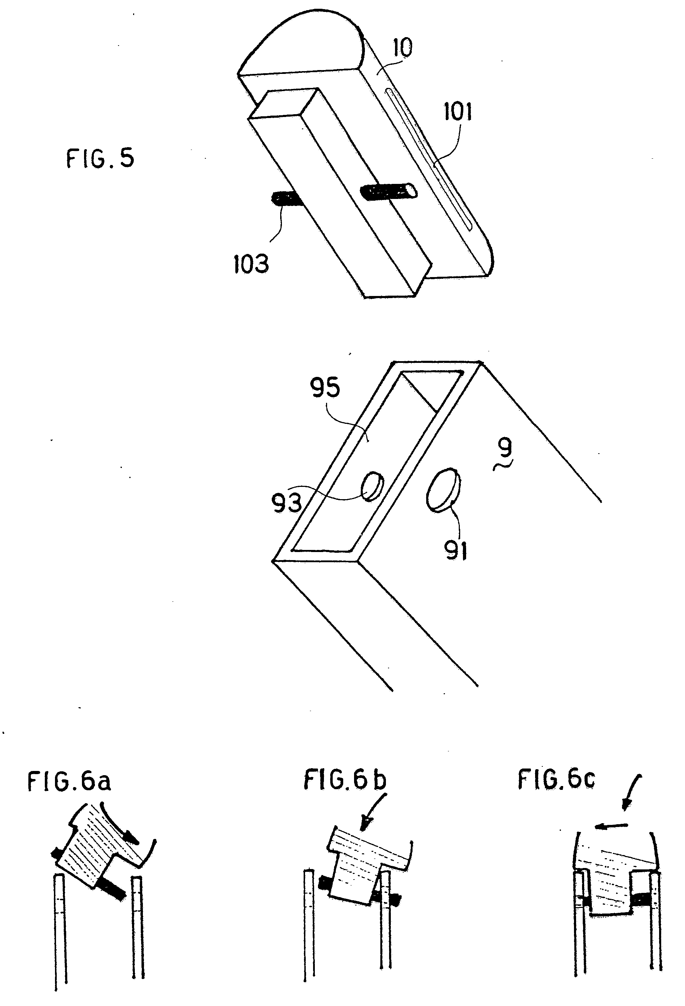

A la figure 5, on a représenté le dispositif permettant la fixation d'une extrémité de la sangle 3 au boîtier 9. Il comporte un élément 10 qui est représenté avant son emboîtement dans une extrémité ouverte 95 du boîtier 9.

L'élément 10 comporte une rainue 101, dans laquelle la sangle 3 peut circuler librement et est muni d'un ergot 103. La zone solide dans laquelle cet ergot est fixé est asymétrique et disposée sur la partie inférieure de l'ergot.

L'extrémité ouverte 95 du boîtier 9 se présente sous la forme d'un tube rectangulaire creux. Dans les deux faces, supérieure et inférieure, deux orifices permettent le passage de l'ergot. L'orifice 91 de la face supérieure est plus grande que l'orifice 93 de la face inférieure afin de permettre l'inclinaison de l'ergot avant son passage dans le trou inférieur 93.

L'introduction de l'élément 10 dans l'ouverture 95 du boîtier 9 est représentée schématiquement à la figure 6, sous forme de trois vues 6a, 6b et 6c.

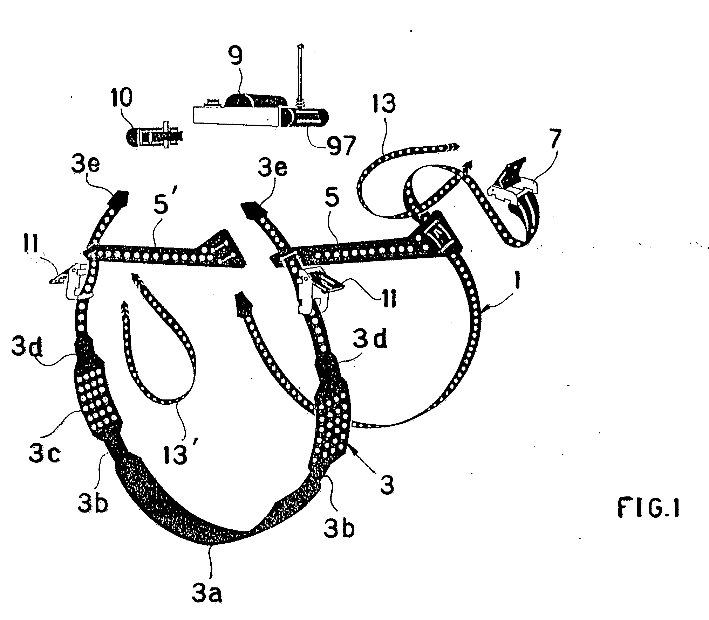

L'extrémité opposée du boîtier 9 est simplement pourvue d'une rainure 97 (voir figure 1) dans laquelle l'autre extrémité de la sangle 3 peut passer librement. En pratique, la fixation se fait en faisant passer en forme de boucle une extrémité de la sangle 3 dans la rainure 101 et l'autre extrémité de la sangle 3 dans la rainure 97 de l'extrémité opposée à l'extrémité 95, l'élément 10 étant solidarisé au boîtier 9 comme indiqué à la figure 6c.

Il est avantageux de compléter cette fixation par un système de verrouillage (non représenté) qui a pour fonction d'obturer l'orifice supérieur 91 et d'empêcher donc le relèvement de l'ergot 103 et dès lors sa sortie de la face inférieure. Les principaux avantages sont l'absence de pièce mobile, la résistance à la traction et la simplicité de manipulation.

Après être passées respectivement dans les rainures 101 et 97, les extrémités des sangles repassent dans les dispositifs de fixation 11 et sont tendus. La tension exercée est mesurée par l'équipement contenu dans le boîtier 9 et qui indique par un signal le moment où une tension correcte est appliquée. Cet équipement de plus peut bien entendu exercer les fonctions décrites dans la publication WO 87/01573 prémentionnée.

Selon une forme d'exécution particulièrement préférée de surveillance du type de celle décrite dans cette publication, le système électronique de surveillance est enclenché par un interrupteur magnétique dont la fonction "contact" se trouve dans le boîtier 9 et la fonction "actuateur" se trouve dans la boucle de verrouillage 10.

Ceci permet, sans devoir utiliser des interrupteurs classiques, d'automatiquement enclencher la fonction surveillance par un dispositif simple, bien protégé et qui ne risque donc pas de détériorations.

La sangle 3 en particulier, mais également les autres sangles, peuvent être conçues de façon à comporter une entaille de rupture de manière que si l'animal s'accroche dans une clôture ou autre sans pouvoir se dégager, une rupture soit possible si l'animal exerce des efforts trop violents qui risqueraient de le blesser. En cas d'une surcharge modérée, une protection automatique est assurée par la partie élastique 3a.

Le dispositif tel que décrit maintient de manière efficace le boîtier de mesure sur l'animal, sans rotation ou translation et il est bien toléré. Il permet une transpiration normale de l'animal, en particulier à cause des multiples perforations qu'il comporte. Le dispositif est ajustable en longueur et adaptable en position. Des réglages de la longueur des sangles avant et arrière, des transversales et des bouclettes sont très facilement réalisables en fonction de la morphologie de l'animal.

Bien qu'on ait décrit une forme d'exécution particulièrement préférée de l'invention, il est bien entendu que de nombreuses variantes de conception, de dimensionnement, de choix de matières sont possibles tout en restant dans le cadre de l'invention. Même en cas d'ouverture accidentelle du verrouillage de l'élément 10 dans le boîtier 9, la sangle avant 1 et les transversales 5 et 5′ retiennent le dispositif sur l'animal. Un signal indiquant cette ouverture peut être alors émis.

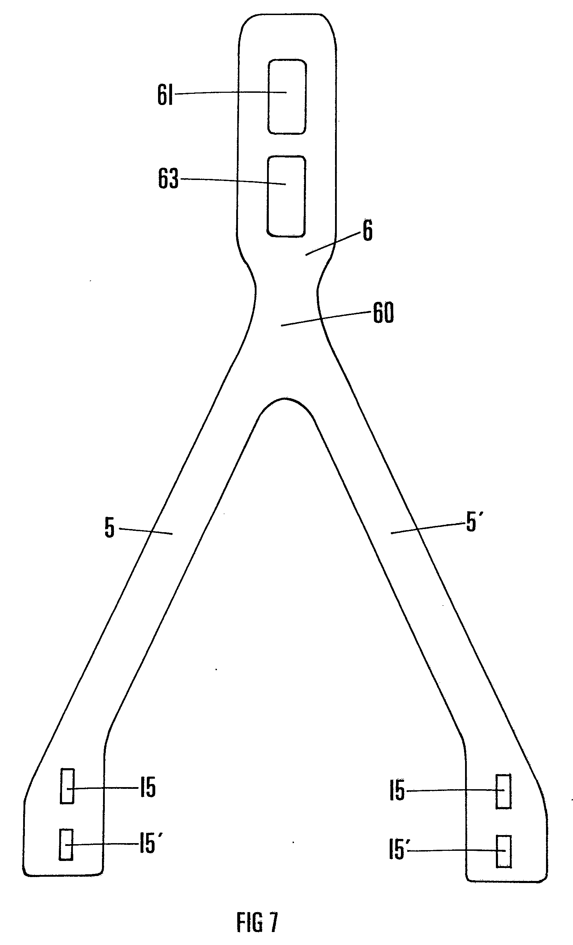

La figure 7 représente une forme particulière d'exécution des sangles transversales supérieures formant un ensemble rejoignant ces deux sangles transversales 5 et 5′ en 60 en une seule patte 6 qui est destinée à être solidarisée au boîtier de mesure 9 et par conséquent à la sangle arrière qui maintient, comme décrit précédemment, ce boîtier de mesure 9 sur l'animal. L'extrémité de la patte de jonction des deux sangles transversales comporte deux découpes rectangulaires 61 et 63 dans le sens longitudinal de la patte 6. La découpe rectangulaire 63 présente une longueur équivalente pratiquement à la dimension de largeur du boîtier de mesure tandis que la découpe rectangulaire 61 est plus longue. Les deux découpes 61 et 63 sont séparées par un espace de dimension correspondant à l'épaisseur du boîtier 9. Les deux extrémités opposées à la patte 6 des sangles 5 et 5′ sont identiques à ce qui a été décrit précédemment.

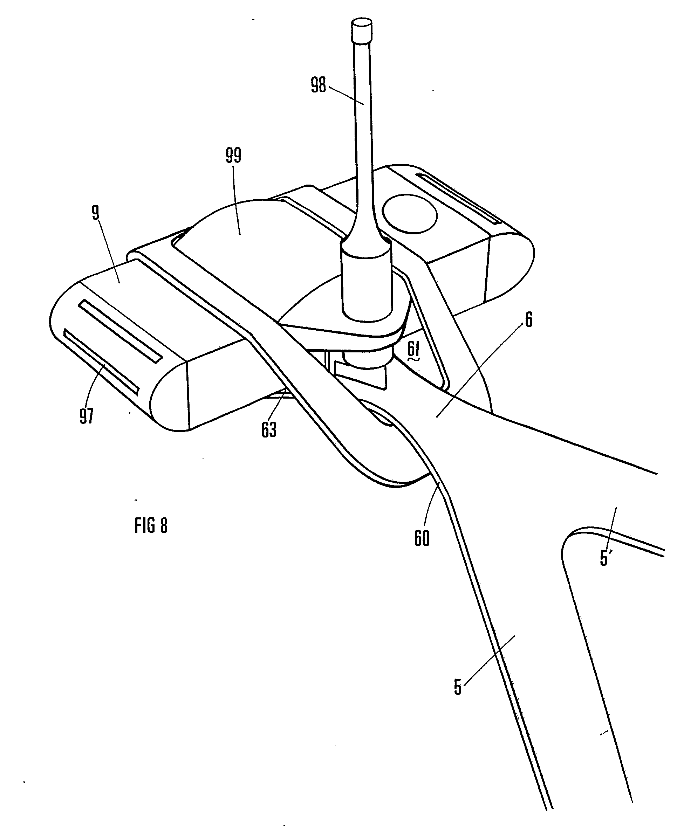

La figure 8 représente le moyen de liaison de la patte 6 de jonction des sangles transversales 5 et 5′ au boîtier de mesure 9. De préférence, ledit boîtier comporte une partie supérieure bombée ou dôme 99 et est équipé d'une antenne. L'antenne 98 et le dôme 99 du boîtier de mesure 9 sont introduits dans l'ouverture faite par la découpe 61 de la patte 6. Aprés avoir enveloppé à l'aide de la patte 6 de la sangle le boîtier de mesure 9 de telle manière que ce boîtier 9 soit en contact avec l'animal au travers de la découpe 63, on introduit l'autre partie formée des deux sangles transversales 5 et 5′ se rejoignant en 60 dans la découpe 61. Cette manière de procéder permet de relier les sangles transversales 5 et 5′ à la sangle arrière 3 par l'intermédiaire du boîtier en fixant cette sangle dans les rainures 97 et 101 comme indiqué précédemment. Ceci préserve l'animal de blessures qui pourraient être occasionnées par le boîtier.

## Revendications

1. Dispositif à sangles destiné à des animaux caractérisé en ce qu'il est constitué par une sangle avant (1) et une sangle arrière (3) comportant au moins partiellement une zone élastique formant ceinture en entourant l'abdomen de l'animal et par au moins une sangle transversale supérieure (5), et de préférence deux sangles transversales supérieures (5 et 5′), reliant la sangle avant (1) et la sangle arrière (3).

2. Dispositif selon la revendication 1 caractérisé en ce que chaque sangle transversale (5 et 5′) est conçue à chaque extrémité pour comporter un moyen tel qu'une boucle ou une découpe (15) lui permettant de recevoir par glissement la sangle arrière (3) et la sangle avant (1), lesdites sangles transversales (5 et 5′) étant prévues pour former un angle déterminé par rapport aux sangles arrières (3) et avant (1) quand l'animal en est équipé et sont pourvues de moyens pour leur maintien en place.

3. Dispositif selon la revendication 2 caractérisé en ce que des moyens pour le maintien en place des sangles transversales (5) sont constitués par des découpes (15 et 15′) dans lesquelles passe la sangle avant (1) ou arrière (3) et au moins une languette de positionnement découpée dans la matière de la sangle transversale (5).

4. Dispositif selon l'une quelconque des revendications précédentes caractérisé en ce que deux sangles transversales supérieures (5 et 5′) se rejoignent en une patte de jonction (6).

5. Dispositif selon la revendication 4 caractérisé en ce que la patte (6) comporte deux découpes (61 et 63) dans le sens longitudinal de ladite patte (6) qui permettent de maintenir un boîtier de mesure (9), en enveloppant ce boîtier (9) par ladite patte (6).

6. Dispositif selon l'une quelconque des revendications précédentes caractérisé en ce qu'il est prévu sur la sangle avant (1), deux petites sangles (13 et 13′) en forme de bouclettes destinées à entourer chacune une patte avant de l'animal et contribuant au maintien en place de l'ensemble des sangles lors de leur utilisation sur l'animal.

7. Dispositif selon la revendication 6 caractérisé en ce que les sangles formant bouclettes (13 et 13′) présentent une terminaison en sapin qui est engagée dans un des trous de la sangle avant (1).

8. Dispositif selon l'une quelconque des revendications précédentes caractérisé en ce que la sangle arrière (3) est destinée à transmettre l'activité musculaire vers le dispositif de mesure contenu dans un boîtier (9) qui est pourvu à deux extrémités de moyens de fixation pour les deux extrémités de la sangle arrière (3), cette sangle étant équipée également de dispositifs de réglage de tension (11).

9. Dispositif selon la revendication 8 caractérisé en ce que ladite sangle arrière (3) est constituée sur sa partie centrale (3a) d'une bande élastique, tandis que les deux extrémités (3c) ne sont que très faiblement déformables élastiquement.

10. Dispositif selon la revendication 9 caractérisé en ce qu'une zone de transition (3b) entre la partie élastique médiane (3a) et les extrémités (3c, 3d et 3e) est elle-même non élastique mais de largeur moindre que les parties adjacentes (3c et 3a).

11. Dispositif selon la revendication 10 caractérisé en ce que les parties destinées à s'appliquer sur le flanc de l'animal (3c) sont pourvues de perforations qui assurent l'évaporation de la transpiration de l'animal.

12. Dispositif selon l'une quelconque des revendications 9 à 11 caractérisé en ce que la sangle arrière (3) ou tout autre élément du dispositif à sangles est constituée d'une bande de matière du type EPDM (qualité alimentaire) vulcanisé ou similaire qui est renforcée dans les parties pratiquement non élastiques (3b, 3c et 3d), par une toile textile incorporée dans le polymère au cours de la fabrication de la matière qui sera découpée ensuite.

13. Dispositif selon l'une quelconque des revendications 1 à 12 caractérisé en ce que l'ensemble des sangles est pourvu de perforations.

14. Dispositif selon l'une quelconque des revendications 8 à 13 caractérisé en ce que le dispositif de mesure (9) qui est destiné à analyser en usage l'activité musculaire de l'animal, permet également de mesurer la tension dans la sangle et peut servir à indiquer par un signal, un réglage correct ou un réglage incorrect (aussi bien vers le haut que vers le bas) de cette tension et ceci tant lors de la mise en place qu'après mise en place.

15. Dispositif selon l'une quelconque des revendications précédentes caractérisé en ce qu'au moins une desdites sangles comporte une entaille de rupture ou tout autre dispositif de sécurité mécanique.

16. Dispositif selon l'une quelconque des revendications précédentes caractérisé en ce que le montage des différentes sangles permet une adaptation ou un réglage en fonction de la morphologie de l'animal.

17. Dispositif selon l'une quelconque des revendications précédentes caractérisé en ce qu'il est équipé d'un dispositif de surveillance de l'animal qu'il est destiné à équiper.

18. Dispositif selon la revendication 17 caractérisé en ce qu'il comporte un dispositif électronique de surveillance qui est enclenché par un interrupteur magnétique dont la fonction contact se trouve dans un boîtier (9) et la fonction actuateur dans une boucle de verrouillage (10) destinée à être reçue dans ledit boîtier (9).

19. Dispositif selon la revendication 18 caractérisé en ce que la boucle de verrouillage (10) comporte une rainure (101) dans laquelle la sangle arrière (3) peut circuler librement, cette boucle de verrouillage (10) étant munie d'un ergot (103), la zone solide dans laquelle cet ergot est fixé étant asymétrique et disposée sur la partie inférieure de l'ergot, tandis que le boîtier (9) qui est destiné à recevoir cet ergot comporte une extrémité ouverte (95) se présentant sous la forme d'un tube rectangulaire creux, dont les deux faces, supérieure et inférieure, sont munies de deux orifices permettant le passage de l'ergot, l'orifice (91) sur la face supérieure étant plus grand que l'orifice (93) de la face inférieure afin de permettre l'inclinaison de l'ergot avant son passage dans le trou inférieur (93)

20. Dispositif selon la revendication 19 caractérisé en ce qu'un système de verrouillage permet d'obturer l'orifice supérieur (91) en vue d'empêcher le relèvement de l'ergot (103) et dès lors sa sortie de la face inférieure de l'extrémité ouverte (95) du boîtier (9).

FIG.1

# FIG.2

15'

17

15

5

3

# FIG. 3

FIG.4

FIG. 5

10

101

103

95

93

91

9

FIG. 6a

FIG. 6 b

FIG. 6c

FIG 7

FIG 8

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4 ) |
|---|---|---|---|
| A,D | US-A-4676198 (MURRAY) <br> * abrégé; figures 4, 5, 6 * <br> --- | 1-3 | A01K27/00 <br> A01K29/00 <br> A61D17/00 |
| A,D | WO-A-8701573 (AGRITRONICS) <br> * abrégé * <br> --- | 8 | A61B5/08 <br> A61B5/03 |
| A | BE-A-462443 (MARLAIR) <br> * page 3, alinéa 4 - page 4, alinéa 1; figures 1, 2 * <br> --- | 1, 5 | |
| A | DE-B-1124631 (MINNER) <br> --- | | |
| A | FR-A-2353225 (GOUILLEUX) <br> --- | | |
| A | US-A-3483861 (TIEP) <br> --- | | |
| A | GB-A-2157937 (SMITH) <br> ----- | | |

DOMAINES TECHNIQUES
RECHERCHES (Int. Cl.4 )

A61B
A61D
A01K

Le présent rapport a été établi pour toutes les revendications

| Lieu de la recherche | Date d'achèvement de la recherche | Examinateur |
|---|---|---|
| LA HAYE | 17 AVRIL 1989 | VON ARX V.U. |

EPO FORM 1503 03.82 (P0402)